Europäisches Patentamt

European Patent Office

Office européen des brevets

(19)

(11) Numéro de publication: **0 373 070**
**A1**

## (12) DEMANDE DE BREVET EUROPEEN

(21) Numéro de dépôt: 89403389.3

(22) Date de dépôt: 06.12.89

(51) Int. Cl.⁵: **A61K 39/21, C12N 15/48**

(30) Priorité: **06.12.88 FR 8815940**

(43) Date de publication de la demande:
**13.06.90 Bulletin 90/24**

(84) Etats contractants désignés:
**AT BE CH DE ES FR GB GR IT LI LU NL SE**

(71) Demandeur: **CENTRE NATIONAL DE LA RECHERCHE SCIENTIFIQUE**
**15, quai Anatole France**
**F-75007 Paris(FR)**

(72) Inventeur: **Madaule, Pascal Jean Jacques**
**3 Impasse de l'Eglise Vauhallan**
**F-91430 Igny(FR)**
Inventeur: **Rossier, Jean Pierre**
**9 rue Parent de Rosan**
**F-75016 Paris(FR)**

(74) Mandataire: **Lemoine, Michel Cabinet**
**Lemoine et Bernasconi et al**
**13 Boulevard des Batignolles**
**F-75008 Paris(FR)**

(54) **Procédé de préparation d'une librairie de déterminants antigéniques peptidiques, nouveaux peptides constitués par ou comportant les déterminants antigéniques obtenus et application des peptides, notamment au diagnostic.**

(57) Procédé de préparation d'une librairie de déterminants antigéniques peptidiques continus d'une protéine ou d'une pluralité de protéines exprimée(s) par un organisme ou microorganisme et correspondant à son génome ou à une partie de son génome, procédé dans lequel on fragmente ce génome ou cette partie de génome au hasard en une multitude de petits fragments que l'on exprime ensuite dans un système d'expression, par exemple une levure, ne présentant pas d'homologie avec ledit génome à l'aide d'un vecteur d'expression approprié et on crible la totalité ou la majeure partie des produits d'expression à l'aide d'anticorps, monoclonaux ou non.

Le procédé est applicable notamment à la constitution d'une librairie de peptides portant les déterminants antigéniques d'un virus tel que HIV, et en particulier HIV1.

Xerox Copy Centre

## Procédé de préparation d'une librairie de déterminants antigéniques peptidiques, nouveaux peptides constitués par ou comportant les déterminants antigéniques obtenus et application des peptides, notamment au diagnostic.

La présente invention a trait à un procédé de préparation d'une librairie de déterminants antigéniques peptidiques, librairie composée de peptides dont certains, au moins, présentent un déterminant antigénique similaire ou identique à un déterminant antigénique d'un produit exprimé par un microorganisme ou une cellule.

L'invention est applicable, notamment, à la constitution d'une librairie de peptides portant les déterminants antigéniques d'un virus tel que HIV et, en particulier, HIV1.

L'une des principales difficultés rencontrées dans le domaine de l'immunologie consiste à identifier et à localiser, dans une protéine exprimée par un organisme vivant, notamment un microorganisme, ou à partir d'un virus, le ou les quelques déterminants antigéniques ou épitopes constitués d'une séquence continue d'acides aminés. Généralement, ces épitopes continus sont étudiés par synthèse peptidique chimique ainsi que par des analyses informatiques de séquences (1). Ces procédés ont permis de caractériser quelques épitopes continus pour le virus HIV1 mais ils ne sont guère faciles à utiliser pour une recherche et une caractérisation systématique de tous les épitopes d'une, voire de la totalité des protéines exprimées par un génome, par exemple génome de HIV1.

La présente invention se propose de remédier aux inconvénients de l'art antérieur et de fournir un procédé permettant de produire et de détecter, sans biais, tout peptide antigénique de l'organisme étudié, notamment de virus, y compris HIV, sans devoir rechercher, pour chaque peptide, un vecteur particulier.

Un autre objectif de l'invention est de fournir un procédé permettant de produire, grâce à des systèmes d'expression efficaces et stables, des peptides utilisables, sans nécessiter de purification, pour des tests ou des diagnostics.

Un autre objectif de l'invention est de fournir des peptides antigéniques dont les constantes d'affinité correspondent à celles d'épitopes conformationnels et sont similaires à celles des épitopes naturels.

Un autre objectif de l'invention est de fournir un procédé de préparation d'une librairie de déterminants antigéniques peptidiques continus pour le virus HIV1.

Un autre objectif de l'invention est de fournir un tel procédé qui puisse également être utilisé pour la caractérisation de déterminants antigéniques peptidiques continus pour d'autres organismes ou microorganismes.

Un autre objectif de l'invention est encore de fournir une librairie constituée par ou contenant des épitopes continus de gènes, voire de génomes, de procaryotes, notamment HIV1, ou d'eucaryotes.

Les épitopes caractérisés par le procédé selon l'invention peuvent être ensuite utilisés, notamment par voie de synthèse peptidique chimique ou biologique, pour préparer des substances de tests ou de diagnostic, destinées notamment à la recherche d'anticorps, pour faciliter la recherche d'anticorps monoclonaux spécifiques d'épitopes, ou pour préparer des vaccins.

L'invention a pour objet un procédé de préparation d'une librairie de déterminants antigéniques peptidiques continus d'une protéine ou d'une pluralité de protéines exprimée(s) par un organisme ou microorganisme et correspondant à son génome ou à une partie de son génome, par exemple un ou plusieurs gènes, caractérisé en ce que,

- on fragmente ce génome ou cette partie de génome au hasard en une multitude de petits fragments pour constituer une librairie de fragments d'acide nucléique,

- on exprime lesdits fragments dans un système d'expression ne présentant pas d'homologie avec ledit génome ou ladite partie de génome, à l'aide d'un vecteur d'expression approprié,

- on crible la totalité ou la majeure partie des produits d'expression à l'aide d'anticorps, monoclonaux ou non,

- et, éventuellement, on localise, dans une carte, les fragments reconnus lors du criblage et/ou on détermine leurs séquences.

La première étape de fragmentation peut porter soit sur un génome entier, par exemple le génome de HIV1, soit sur une partie de génome suffisamment grande pour comporter une grande partie ou la totalité du gène codant pour une protéine dont on cherche à caractériser les épitopes. Si le matériau génétique initial est sous forme d'ARN, on utilise sa transcription ADN obtenue soit par voie naturelle, soit par un procédé usuel de transcription inverse.

Par petits fragments dans le sens de la présente invention, on entend des fragments d'ADN obtenus au hasard et ayant une taille de quelques dizaines à quelques centaines de paires de bases et qui, de préférence, pour la majorité d'entre eux, n'est pas supérieure à 50 ou 100 à 150 ou 200 pb (paires de bases) et, de préférence, de l'ordre de 50 pb.

La fragmentation du matériau génétique traité peut être effectuée, de préférence, par digestion partielle enzymatique, par exemple, par DNAseI. Elle peut également être effectuée par toute autre méthode de fragmentation permettant de réaliser au hasard une multitude de petits fragments, par exemple par sonication. Dans le cas de la fragmentation de l'ADN proviral de HIV1, on peut obtenir $4 \times 10^4$ clones environ.

Le système d'expression préféré, notamment pour la constitution de librairies virales, est une levure, de préférence Saccharomyces cerevisiae. Ce système est préféré à des systèmes bactériens, car il permet de nombreuses modifications post-traductionnelles des peptides exprimés et, en outre, il ne comporte pas d'éléments toxiques pour les mammifères.

Un procédé d'expression de protéines dans cette levure utilisant le gène du facteur alpha, a déjà été décrit (2-5). La biosynthèse (5) du facteur alpha, qui est l'une des deux hormones peptidiques impliquées dans la reconnaissance sexuelle de la levure, inclut la formation d'un grand précurseur dont la partie N-terminale est vraisemblablement impliquée dans la reconnaissance d'un système de sécrétion alors que la moitié C-terminale contient quatre domaines hormonaux séparés par des sites de clivage spécifiques Lys-Arg. Lorsqu'un ADN étranger est inséré en aval du premier site de clivage, la protéine recombinante peut être sécrétée dans le milieu de culture, pratiquement vierge de résidus de levure, avec des glycosylations sur les sites N- et même O-.

L'étape d'insertion des fragments dans la levure peut être effectuée de toute façon en soi connue, en utilisant, par exemple, un vecteur plasmidique.

Une fois la librairie plasmidique constituée, la culture cellulaire, telle que Saccharomycescerevisiae, est transfectée, puis clonée. On peut alors procéder au criblage des clones à l'aide d'un lot de sérums et/ou d'anticorps selon des procédés usuels, pâr exemple selon Lyons et Nelson (13).

Pour caractériser les séquences des inserts reconnus lors du criblage, on peut reprendre les vecteurs de la colonie du clone d'expression, par exemple levure, et récupérer les inserts qui peuvent être sous-clonés et séquencés par l'un quelconque des procédés usuels de séquençage. La reconnaissance de fragments ayant réagi et se chevauchant partiellement simplifie cette étape.

L'application du procédé selon l'invention au génome de HIV1 a permis de caractériser six épitopes continus dont deux n'avaient jamais été décrits. La présente invention a également pour objet les nouveaux peptides contenant ces épitopes ou constitués par eux, et notamment un épitope dans l'endonucléase (pol), deux épitopes de gp 25 et un épitope de gp41.

D'autres avantages et caractéristiques de l'invention apparaîtront à la lecteur de la description suivante, faite à titre d'exemple non limitatif et se référant au dessin annexé dans lequel :

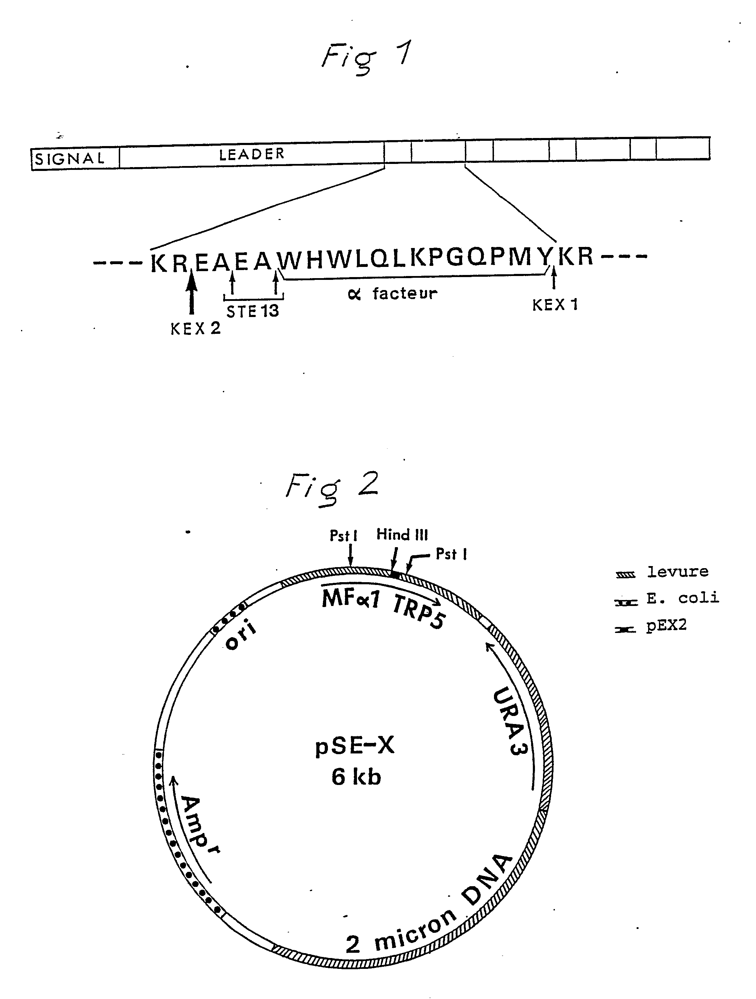

- la figure 1 représente la structure générale du précurseur du facteur alpha de Saccharomyces cerevisiae,

- la figure 2 représente une carte du plasmide d'insertion et de transfection PSE-X,

- la figure 3 représente le cadre de lecture au site de clonage de ce plasmide,

- la figure 4 représente les chevauchements de séquence de plusieurs inserts définissant des épitopes,

- la figure 5 représente les réactions comparées de différents sérums et anticorps détectant les épitopes exprimés dans la levure,

- la figure 6 représente des courbes de sécrétion de peptides recombinants de plusieurs clones reconnus par l'un des sérums par test ELISA,

- la figure 7 représente des courbes de sécrétion et de durée de croissance par mesure de la turbidité d'un clone reconnu par l'un des sérums.

## 1. CONSTITUTION D'UNE LIBRAIRIE DE PETITS FRAGMENTS DE HIV1.

On a utilisé comme matériau de départ le plasmide pBT-1 qui est un plasmide pUC-18 (pUC18 est disponible chez International Biotechnologies, Inc., P.O. BOX 9558, 275 Winchester Avenue, New Haven, CT 06535 USA), intégrant un provirus complet de HIV1, isolant BRU (10). Ce plasmide pBT-1 est accessible dans la collection du Professeur Luc Montagnier ou Pierre Sonigo, Institut Pasteur, 25 rue du Dr Roux 75015 PARIS (France).

Une quantité de 20 μg de pBT-1 a été digérée partiellement par la DNAse I selon le procédé décrit par Anderson (11), les conditions de lyse enzymatique et de purification des fragments par électrophorère en gel d'agarose ayant été adaptées pour obtenir des fragments de l'ordre de 50-150 pb. Les extrémités des fragments obtenus ont été réparées par l'ADN polymérase du phage T4, mais n'ont pas été modifiées par l'adjonction d'ADN synthétique du type linker.

## 2. CONSTRUCTION DU VECTEUR pSE-X.

Ce plasmide, obtenu à partir de pUC18, comporte cinq parties d'ADN étranger, à savoir : un fragment de 600 pb du gène du facteur alpha (MF α 1) contenant le promoteur et le début de la séquence codant pour le gène du facteur alpha jusqu'au premier site de clivage Lys-Arg ; puis un fragment de 35 pb d'un plasmide pEX-2 fournissant un codon stop pour chaque cadre de lecture de (MF α 1) (9);puis un fragment de 400 pb de TRP5 portant des signaux terminateurs de gène (3) ; ensuite le gène URA3 utilisé comme marqueur détectable dans la levure ; enfin, un fragment de l'ADN 2 micron de levure portant un site d'origine de réplication. La région du plasmide pUC18, qui permet le polyclonage au voisinage du site de clivage Hind III, a été éliminée.

Cette construction plasmidique comporte un site de restriction Hind III à la fin du fragment du gène MF α 1.

La construction peut être effectuée de la façon suivante : partant du plasmide pUC18, on insère entre les sites Hind III - BamHI, un fragment Hind III - BmHI de 600 pb du gène du facteur qui porte le promoteur suivi de la séquence codante jusqu'au premier site de clivage Lys -Arg. Ensuite, on insère dans le site NdeI du plasmide, l'origine de réplication 2 microns de la levure puis le gène URA3. Le fragment pvuII - BamHI de 185 pb a été éliminé par restriction. Ce plasmide, mis au point par Allan Myers, Department of Biochemistry and Biophysic, Iowa State University, Ames, IA50011, USA, et dénommé pSE3 (construction non publiée) a alors été modifié par échange du fragment de 90 pb Hind III - PvuII par un fragment de 35 pb Hind III - XbaI du vecteur pEX2 (9), ce qui insère un codon stop pour chaque cadre de lecture du gène du facteur α. Dans le plasmide ainsi modifié, on insère dans le site PvuI de LacZ, le fragment EcoRI-Hind III de 400 pb du vecteur pTRP54 (3) qui contient tous les signaux terminateurs de ce gène dans la levure (3), ce qui donne le vecteur pSE-X. Toutes les liaisons mettant en oeuvre des extrémités d'ADN non compatibles ont été effectuées après traitement préalable à l'enzyme de Klenow ou à la T4 ADN polymérase.

Le plasmide pSE-X a été déposé, dans E. coli, le 1er décembre 1988 sous le numéro I-822 auprès de la CNCM (Collection Nationale de Cultures de Microorganismes, France).

## 3. INSERTION DES FRAGMENTS DANS LE VECTEUR pSE-X.

Les fragments obtenus selon 1. ci-dessus ont été insérés dans la constructions pSE-X à l'emplacement du site de restriction Hind III.

Les différents vecteurs ainsi obtenus ont été utilisés pour transformer une souche de E. Coli (souche SCS1 de la Société Stratagene), ce qui a permis d'obtenir environ 40.000 clones indépendants qui ont été amplifiés. Une analyse au hasard de 12 clones montre que 91 % possédaient un fragment inséré.

## 4. TRANSFECTION DE LA LEVURE.

La souche de Saccharomyces cerevisiae W303-1B (MAT α, leu2, his3, ura3, trp1, ade2) a été utilisée (Slonimsky, CNRS, CGM, avenue de la Terrasse, 91190 Gif sur Yvette, France). Les différentes colonies bactériennes selon 3. ont été réunies et ont permis la préparation de 5 mg d'ADN plasmidique. 10 microgrammes de cet ADN ont été introduits dans la souche de levure précitée en utilisant la procédure au lithium (12).

A titre de contrôle positif, on a également cloné dans le site Hind III de pSE-X un fragment d'ADN pstl-Apal de 587 pb de pBT-1 codant pour les 167 résidus C-terminaux de la protéine p25 et les 28 résidus N-terminaux de la protéine p13 de HIV1. Cette construction a été appelée pGAG.

## 5. CRIBLAGE DE CLONES.

Environ 10.000 clones de levure ont été dispersés sur boîte de Pétri contenant un milieu sélectif (3) pour la persistance de plasmides contenant URA3. Après 30 heures de culture à $30^\circ$C, les levures ont été transférées sur filtre nitrocellulose et lysées in situ (13). La feuille de filtre a été analysée à la manière d'un western blot (procédure au lait écrémé). Toutefois, la plupart des sérums humains ont dû être immunoadsorbés de la façon suivante : de la levure de boulanger (Fould-Springer) a été dispersée dans de l'eau (1 ml d'eau par gramme de levure) et maintenue à $100^\circ$C pendant 20 mn. Le pH a été ajusté à 7,3 et 0,05 volume de tampon 10X PBS a été ajouté. Les sérums ont été dilués dans le lysat de levure au 1/300 et incubés à $4^\circ$C pendant une nuit. Après centrifugation, le surnageant a été utilisé directement pour l'incubation avec les filtres de nitrocellulose. On a utilisé des seconds anticorps couplés à la peroxydase. La diaminobenzidine en présence de nickel (14) a été utilisée pour la coloration jusqu'à ce que le clone de contrôle négatif portant le plasmide pSE-X sans insert devienne légèrement visible.

Le criblage a été effectué à l'aide de : trois sérums, 3, 5 et 6, fournis par l'Institut Pasteur (Paris, France) et provenant de patients qui se sont avérés positifs pour toutes les protéines structurales du virus HIV1 dans une analyse western blot ; un anticorps monoclonal humain, référencé m, provenant d'un volontaire pour un essai de vaccination (12); et trois anticorps monoclonaux de souris référencés a à c et dirigés contre la glycoprotéine gp110 de HIV1.

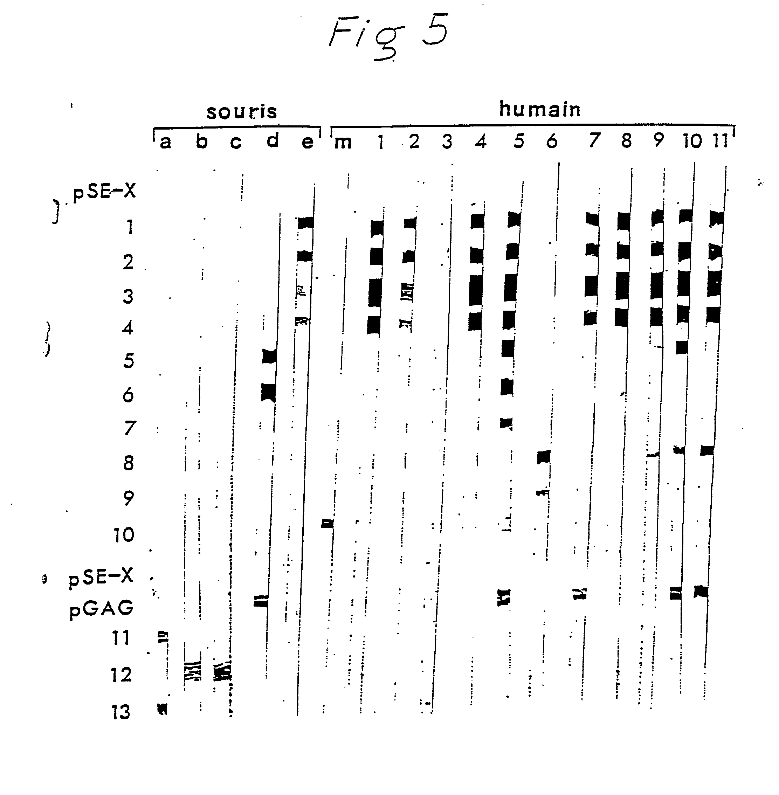

Les résultats apparaissent sur la figure 5, qui a été obtenue comme pour une expérience de criblage, sauf qu'ont été étalés des clones préalablement caractérisés (en ce sens qu'il ont été détectés par des anticorps lors des expériences de criblage, et que les épitopes produits ont été identifiés), chaque clone étant étalé sous forme d'une longue traînée.

La feuille de nitrocellulose, après transfert des protéines, a été découpée perpendiculairement aux traînées, de sorte que chaque bandelette de filtre contenait en zones successives, les protéines (donc les épitopes) des clones de levures étudiées. Ainsi, l'activité de différents anticorps (sérum ou monoclonaux) a pu être aisément comparée.

On a utilisé onze sérums humains, numérotés de 1 à 11, provenant de patients qui se sont avérés positifs pour toutes les protéines structurales du virus HIV1 dans une analyse western blot, l'anticorps monoclonal humain, référencé m, et cinq anticorps monoclonaux de souris référencés de a à e (trois dirigés contre la gp 110 : a, b et c ; un dirigé contre la p25 : d et un contre la gp41: e).

Comme on pouvait s'y attendre, la colonie relative au plasmide de contrôle dépourvu de fragment, pSE-X, n'a été révélée par aucun des anticorps ou sérums. La colonie relative au plasmide pGAG, construite à titre de contrôle positif, a révélé les sérums 5, 7, 10 et 11 ainsi que l'anticorps de souris d. En particulier, le sérum 5 a révélé, de façon très intense, la colonie de contrôle et avait donc été utilisé pour un premier criblage de la librairie. Ce criblage avait révélé sept colonies référencées 1 à 7 et qui ont été analysées.

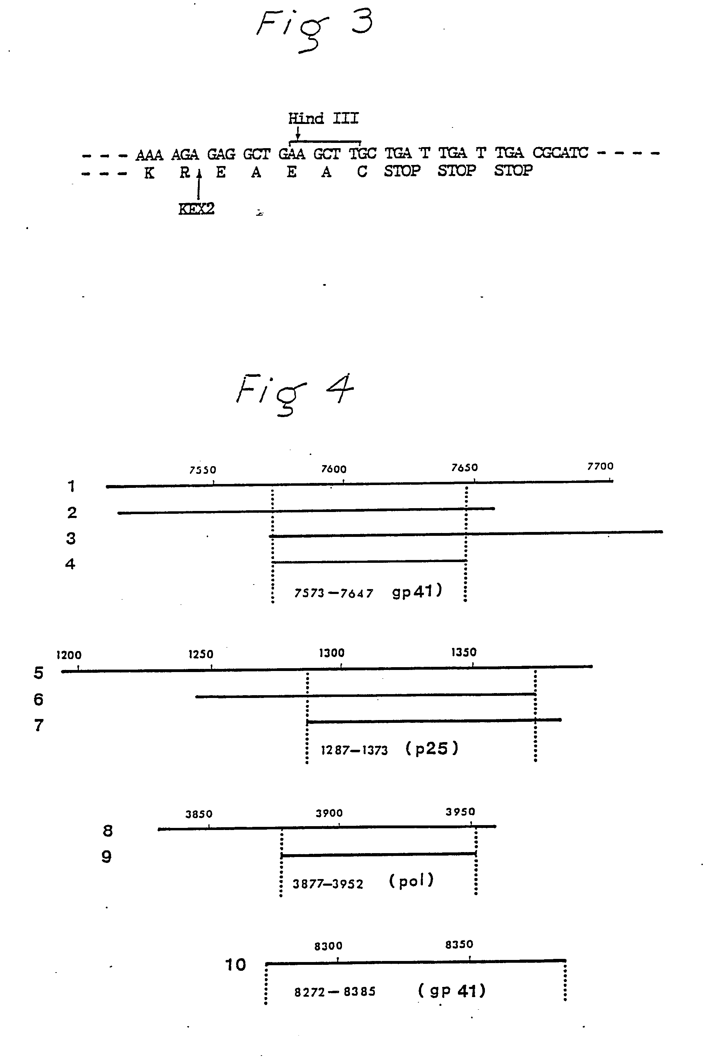

Dans ce but, les plasmides présents dans chaque colonie positive ont été repris dans E. Coli (12). Des fragments PstI portant les inserts ont été sous-clonés dans M13 mp19 et séquencés en utilisant soit une amorce classique M13, soit l'oligonucléotide TGCCAGCATTGCTGCT. Les différentes séquences chevauchantes que l'on obtient ont été représentées sur la figure 4 sur laquelle la numérotation des nucléotides est celle de GENEBANK référence HIVBRUCG. Les clones 1 à 4 ont révélé un premier épitope dans gp41 avec la structure suivante : YLKQQLLGIWGCSKLICTTAVPW. Les clones 5 à 7 ont défini un deuxième épitope dans p25, ayant la structure TETLLVQNANPDCKTILKALGPAATLEEM.

L'épitope de gp41 est vraisemblablement le principal épitope viral actuellement localisé ( 6) et déjà utilisé sous forme de peptide synthétique dans des tests sanguins de seconde génération.

Les deux anticorps monoclonaux de souris d et e (25.7 et 41.1), tous deux induits contre le virus entier, avaient été cartographiés dans ces positions. Ces anticorps monoclonaux ont réagi fortement avec quatre des sept clones et faiblement avec les trois autres. Si cependant on analyse le surnageant de ces trois derniers clones dans un test ELISA, la réaction de ces anticorps monoclonaux s'avère également forte.

Un examen attentif suggère la présence d'un second épitope dans les clones 5, 6 et 7 ayant révélé un épitope dans la région p25. L'anticorps monoclonal 25.7 (d) a révélé, de façon pratiquement identique, les clones 5 et 6. Cependant, le sérum humain 10 a fortement détecté le clone 5 sans détecter le clone 6. En fait, le clone 5, lorsqu'on le compare au clone 6, a inséré un fragment plus long, codant pour dix-sept aminoacides supplémentaires du côté N-terminal et on peut supposer qu'un autre épitope se trouve dans cette région.

Du fait que les sérums 3 et 6 se sont avérés négatifs pour les clones 1 à 7, ils ont été utilisés pour un nouveau criblage de la librairie à la recherche d'autres épitopes. Le sérum 6 a alors détecté deux colonies, référencées 8 et 9, qui, après analyse et séquençage, se sont avérées produire un épitope continu dans l'endonucléase (pol). La structure de cet épitope est : ASDFNLPPVVAKEIVASCDKCQLK. D'autres sérums (9, 10 et 11) ont également détecté cet épitope qui représente donc un épitope majeur de HIV1.

Le criblage à l'aide de l'anticorps monoclonal humain m a révélé un clone, 10. A l'analyse, ce clone produit les trente-huit derniers résidus de gp41 (ce clone utilise effectivement le codon stop du gène env de HIV1 au lieu d'un signal stop provenant du vecteur. Du fait que le peptide synthétique composé des quinze derniers résidus de gp41 ne réagit pas avec l'anticoprs monoclonal m, l'épitope en question n'est probablement pas disposé complètement à l'extrémité C mais un peu en amont. Un épitope majeur dans les cent vingt-neuf derniers résidus de gp41 a déjà été décrit mais non encore cartographié exactement ( 8).

Aucun épitope n'ayant été localisé dans gp110 par les criblages précités, il a été effectué un nouveau criblage à l'aide des anticorps monoclonaux de souris a (110.1), cartographié en 494-517, b (110.4) cartographié en 308-325 et c (110.5) non cartographié. Le monoclonal a a détecté deux clones 11 et 13 alors que b et c ont détecté le même clone 12, ce qui suggère que ces anticorps sont similaires.

## 6. ETUDE DES CARACTERISTIQUES DES SECRETIONS DES CLONES.

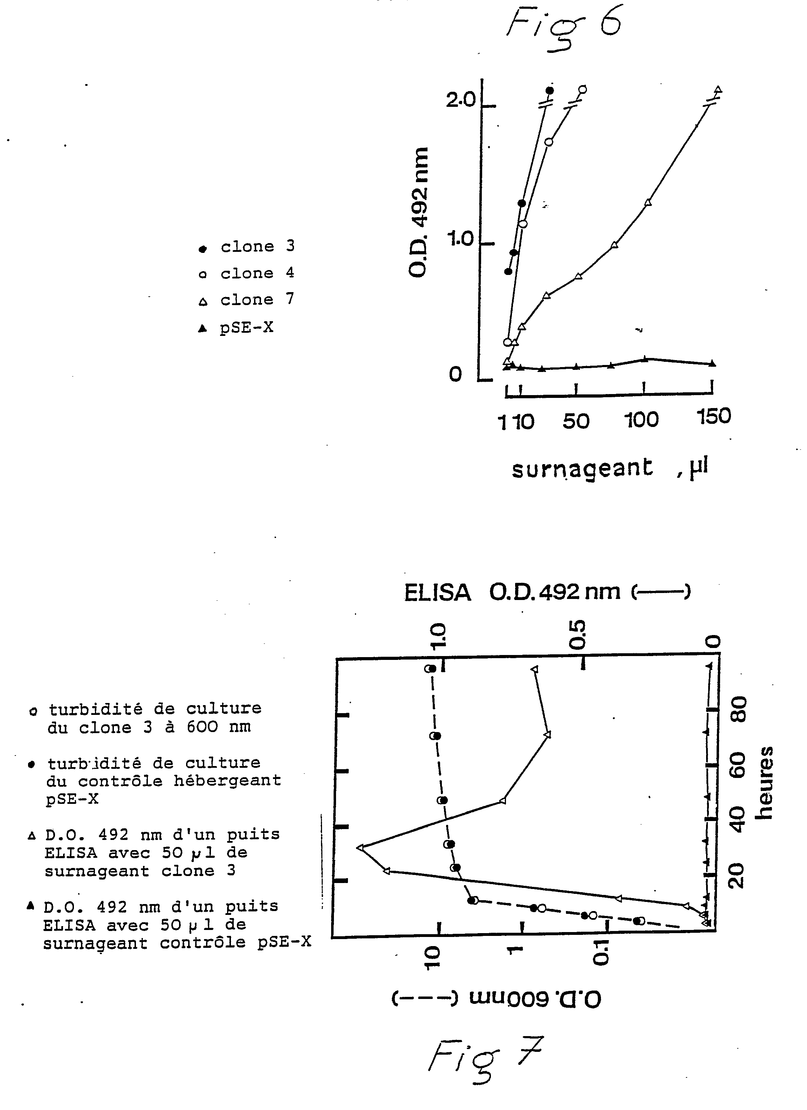

Des plaques ELISA ont été recouvertes de différentes quantités de surnageants de culture des colonies et on a procédé à l'immunodétection des épitopes. Les clones 3, 4 et 7 (voir figure 7), de même que les clones 11, 12 et 13 se sont avérés sécréter de grandes quantités de peptides du fait que quelques microlitres de milieu de culture contiennent suffisamment d'antigène pour entraîner des densités optiques proches de 1 lors de la réaction avec les anticorps. La courbe de sécrétion en fonction du temps a été estimée de façon similaire (figure 7). Ces clones ont donné un pic d'antigène après 30 heures de culture, après quoi on assiste à une lente diminution. Les cinétiques de croissance de ces clones sont pratiquement identiques à celles de la levure de contrôle logeant le plasmide pSE-X dépourvu d'insert, soulignant la très faible toxicité des produits recombinants exprimés.

Tous les clones se sont avérés non maladifs, viables et permanents et expriment bien leurs peptides avec l'épitope bien présenté.

## 7. PRODUCTION DES SUBSTANCES DE TEST OU DE DIAGNOSTIC.

Les clones intéressants décrits ci-dessus, peuvent être cultivés selon les procédés classiques de culture des levures. Le peptide exprimé se retrouve dans le surnageant de culture que l'on débarrasse des cellules et fragments cellulaires, par simple filtration ou centrifugation. Le surnageant constitue la substance de test.

On prépare ainsi des substances comprenant respectivement l'épitope de p41 précité, les épitopes de p25 précités et l'épitope pol précité.

## 8. IMMUNOADSORPTIONS DES SERUMS

Pour débarrasser les sérums des anticorps antilevure, on peut procéder comme pour le criblage des clones. Cependant, on préfère diluer le sérum au moins au 1/50, de préférence au 1/100 ou 1/300 dans une suspension formée à partir de 50 g de levure de boulanger (Fould-Springer) et 50 ml de surnageant de culture produit par la levure contenant le même vecteur d'expression que les levures productrices des peptides mais dépourvu d'insert. La suspension est amenée à 100°C pendant 30 mn. Le pH est ajusté à 7,3 ou 7,4 avec NaOH. Cette suspension se conserve au moins pendant 6 mois à -20°C.

Le sérum dilué dans la suspension est agité doucement une nuit à environ + 4°C, puis les levures sont éliminées par centrifugation brève. Ces sérums dilués se conservent quelques jours à +4°C.

Les sérums dilués peuvent bien entendu être utilisés pour screener une librairie d'expression dans la levure.

## 9. DIAGNOSTICS.

Le sérum du patient est dilué dans la suspension et traité selon 8. ci-dessus.

On enduit des cupules de plaques de test ELISA avec les surnageants respectifs préparés selon 7. Une ou plusieurs autres cupules sont enduites, pour le contrôle, avec un surnageant de culture de levure non productrice de peptides, et, de préférence, transfectée par le vecteur dépourvu d'insert. Les réactions immunologiques, de type ELISA, sont ensuite effectuées de façon usuelle. La différence entre l'intensité de la réaction dans la cupule de test et celle de la cupule de contrôle (souvent très faible) traduit la positivité de la réaction.

Les affinités relevées sont bien plus importantes que celles relevées pour des tests connus basés sur des peptides synthétiques et sont comparables à celles des épitopes naturels, prouvant la similitude conformationnelle des épitopes exprimés conformément à l'invention.

## REFERENCES

1. Westhof, E. et al. Nature 311, 123-126 (1984).

2. Ernst, J.F. Mermod, J.J., DeLamarter, J.F., Mattaliono, R.J. & Moonen, P. Biotechnology 5, 831-834 (1987).

3. Miyajima, A., Bond, M.W., Otsu, K., Arai, K. & Arai, N. Gene 37, 155-161 (1985).

4. Brake, A.J. et al. Proc. Natl. Acad. Sci. USA 81, 4642-4646 (1984).

5. Sterne, R.E., Fuller, R.S. & Thorner, J. Ann. Rev. Physiol. 50, 345-362 (1988).

6. Gnann, J.W., Nelson, J.A. & Oldstone, M.B., J. Virol. 61, 2639-2641 (1987).

7. Zagury, D. et al. Nature 332, 728-731 (1988).

8. Windheuser, M.G. & Wood, C. Gene 64, 107-119 (1988).

9. Stanley, K.K. & Luzio, J.P. EMBO J. 3, 1429-1434 (1984).

10. pBT-1 Luc Montagnier, Pierre Thiollais, et Marc Alizon (non publié) ; le plasmide pBT a le même insert que le phage J19 = Alizon M. et al. Nature 312, 757-760 (1984) ; séquence des nucléotides disponibles dans GENEBANK sous le matricule HIVBRUCG.

11. Anderson, S. Nucleic Acids. Res. 9, 3015-3026 (1981).

12. Sherman, F., Fink, G.R. & Hicks, J.B. in Laboratory Course Manual for Methods in Yeast Genetics (Cold Spring Harbor Laboratory, Cold Spring Harbor, New York, 1986).

13. Lyons, S. & Nelson, N. Proc. Natl. Acad. Sci. USA 81, 7426-7430 (1984).

14. De Blas, A.L. & Cherwinski, A.M. Anal. Biochem. 133, 214-219 (1983).

**Revendications**

1. Procédé de préparation d'une librairie de déterminants antigéniques peptidiques continus d'une protéine ou d'une pluralité de protéines exprimée(s) par un organisme ou microorganisme et correspondant à son génome ou à une partie de son génome, caractérisé en ce que,
- on fragmente ce génome ou cette partie de génome au hasard en une multitude de petits fragments pour constituer une librairie de fragments d'acide nucléique,
- on exprime lesdits fragments dans un système d'expression ne présentant pas d'homologie avec ledit génome ou ladite partie de génome, à l'aide d'un vecteur d'expression approprié,
- et on crible la totalité ou la majeure partie des produits d'expression à l'aide d'anticorps, monoclonaux ou non.

2. Procédé selon la revendication 1, caractérisé en ce que l'on exprime lesdits fragments dans un système de levure.

3. Procédé selon la revendication 2, caractérisé en ce que l'on utilise Saccharomyces cerevisiae, notamment dans le gène du facteur α.

4. Procédé selon la revendication 3, caractérisé en ce que l'on insère les fragments dans le vecteur plasmidique ayant la construction décrite sous pSE-X, à l'emplacement du site de restriction Hind III.

5. Procédé selon l'une des revendications 1 à 4, caractérisé en ce que l'on séquence les fragments reconnus lors du criblage.

6. Procédé selon l'une des revendications 1 à 5, caractérisé en ce qu'on l'applique au génome entier d'un virus.

7. Procédé selon la revendication 6, caractérisé en ce que le virus est HIV1.

8. Procédé selon l'une des revendications 1 à 7, caractérisé en ce que l'on effectue la fragmentation dans des conditions conduisant à des fragments ayant en majorité une taille qui n'est pas supérieure à 50 pb.

9. Procédé selon l'une des revendications 1 à 7, caractérisé en ce que l'on effectue la fragmentation dans des conditions conduisant à des fragments ayant en majorité une taille qui n'est supérieure à 200 pb.

10. Procédé selon la revendication 9, caractérisé en ce que la fragmentation est faite dans des conditions conduisant à des fragments ayant en majorité une taille qui n'est pas supérieure à 150 pb.

11. Procédé selon l'une des revendications 9 et 10, caractérisé en ce que l'on produit des fragments ayant en majorité une taille de 100 à 150 ou 200 pb.

12. Procédé selon l'une des revendications 8 à 11, caractérisé en ce que l'on utilise la DNAse I pour effectuer la fragmentation.

13. Vecteur plasmidique pSE-X.

14. Nouveaux peptides incluant ou contenant un épitope détecté par le procédé selon l'une quelconque des revsendications 1 à 12.

15. Nouveaux peptides selon la revendication 14, contenant ou composés par au moins un épitope continu de HIV1.

16. Nouveaux peptides selon la revendication 15, contenant ou composés par l'épitope de l'endonucléase (pol).

17. Nouveaux peptides selon la revendication 15, contenant ou composés par un épitope de gp41.

18. Nouveaux peptides selon la revendication 15, contenantou composés par un épitope de p25.

19. Nouveaux peptides selon l'une des revendications 17 et 18, caractérisés en ce qu'ils sont exprimés par le procédé selon l'une des revendications 8 à 12.

20. Procédé de production des peptides selon l'une des revendications 14 à 19, caractérisé en ce que l'on cultive le système de levure, notamment S. cerevisiae, notamment dans le gène du facteur α et en ce que l'on recueille les peptides dans le surnageant de culture.

21. Procédé selon la revendication 20, caractérisé en ce que le système de levure possède un insertsous pSE-X.

22. Procédé de production de substances de test ou de diagnostic, notamment dans les infections à HIV, caractérisé en ce que l'on utilise un ou des peptides obtenus par le procédé selon la revendication 20 ou 21.

23. Substances de test ou de diagnostic, caractérisées en ce qu'elles comportent un ou des peptides selon l'une quelconque des revendications 14 à 19.

24. Procédé de test ou de diagnostic mettant en oeuvre un sérum et un ou plusieurs peptides ou substances selon l'une des revendications 14 à 19, obtenus par le procédé selon l'une des revendications 20 à 22, caractérisé en ce que, pour débarrasser le sérum des anticorps anti-levure, le sérum est dilué dans une suspension contenant de la levure et un surnageant de culture delevure contenant le même vecteur d'expression que les levures productrices de peptides mais dépourvu d'inserts.

25. Procédé de test ou de diagnostic, caractérisé en ce que l'on effectue des réactions immunologiques comparatives, de préférence du type ELISA, d'une part, avec une substance de test exprimée dans la levure selon la revendication 23, d'autre part, avec un surnageant de contrôle provenant d'une culture de levure sans vecteur d'expression et/ou avec le même vecteur d'expression que les levures productrices de peptides mais dépourvu d'inserts.

26. Application des peptides selon l'une quelconque des revendications 14 à 19, à la préparation d'un vaccin.

*Fig 1*

--- K R E A E A W H W L Q L K P G Q P M Y K R ---

KEX 2
STE 13
α facteur
KEX 1

*Fig 2*

pSE-X
6 kb

legende: levure, E. coli, pEX2

# Fig 3

Hind III

- - - AAA AGA GAG GCT GAA GCT TGC TGA T TGA T TGA CGCATC - - - -
- - - K   R   E   A   E   A   C   STOP   STOP   STOP

KEX2

# Fig 4

1
2
3
4

7550   7600   7650   7700

7573−7647   gp41)

5
6
7

1200   1250   1300   1350

1287−1373   (p25)

8
9

3850   3900   3950

3877−3952   (pol)

10

8300   8350

8272 − 8385   (gp 41)

# Fig 5

Fig 6

O.D. 492 nm

2.0

1.0

0

● clone 3
○ clone 4
△ clone 7
▲ pSE-X

1 10    50    100    150

surnageant , µl

---

ELISA O.D. 492 nm (———)

1.0    0.5    0

○ turbidité de culture
  du clone 3 à 600 nm

● turbidité de culture
  du contrôle hébergeant
  pSE-X

△ D.O. 492 nm d'un puits
  ELISA avec 50 µl de
  surnageant clone 3

▲ D.O. 492 nm d'un puits
  ELISA avec 50 µl de
  surnageant contrôle pSE-X

20    40    60    80    heures

10    1    0.1

O.D. 600nm (– – –)

Fig 7

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | US-A-4753873 (GERALD A.B.)<br>* colonne 4 * | 1-26 | A61K39/21<br>C12N15/48 |
| X | EP-A-253701 (INSTITUT PASTEUR)<br>* colonne 17 *<br>* colonnes 22 - 27 * | 1-26 | |
| X | EP-A-273716 (THE UNITED STATES OF AMERICA)<br>* revendications 1-13 * | 1-26 | |
| A | EP-A-185444 (CENTOCOR)<br>* pages 17 - 18 *<br>* pages 23 - 24 * | 1-26 | |
| A | EP-A-199301 (HOFFMANN LA ROCHE)<br>* colonnes 11 - 17 * | 1-26 | |
| A | EP-A-276591 (TRANSGENE ET AL.)<br>* le document en entier * | 1-26 | |
| A | Vaccine<br>vol. 5, no. 2, juin 1987, GB<br>pages 90 - 101; P.J.Barr:<br>"Antigenicity and immunogenicity of domains of the human immunodeficiency virus....."<br>* le document en entier * | 1-26 | DOMAINES TECHNIQUES RECHERCHES (Int. Cl.5)<br><br>A61K<br>C12N<br>C12P |

Le présent rapport a été établi pour toutes les revendications

| Lieu de la recherche | Date d'achèvement de la recherche | Examinateur |
|---|---|---|
| BERLIN | 13 FEVRIER 1990 | AVEDEKIAN P.F. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)